# EUROPÄISCHE PATENTSCHRIFT

(11) **EP 0 486 978 B1**
(45) Veröffentlichungstag und Bekanntmachung des Hinweises auf die Patenterteilung: **06.03.1996**
(21) Anmeldenummer: 91119580.8
(22) Anmeldetag: 16.11.1991
(51) Int. Cl.: G01N 33/50, G01N 33/53, G01N 33/80, G01N 21/82

(54) **Verfahren und Vorrichtung zum quantitativen Auswerten von Agglutinationsreaktionen**
Method and device for quantitative evaluation of agglutination reactions
Méthode et dispositif pour évaluation quantitative de réactions d'agglutination

(30) Priorität: 23.11.1990 DE 4037245
(43) Veröffentlichungstag der Anmeldung: 27.05.1992
(73) Patentinhaber: BEHRINGWERKE Aktiengesellschaft, D-35001 Marburg (DE)
(72) Erfinder: Meller, Paul, Dr., W-6070 Langen (DE); Scheuermann, Susanne, W-6380 Bad Homburg (DE); Zimmermann, Matthias, W-6231 Sulzbach (DE)

(56) Entgegenhaltungen:
- EP-A- 0 107 580
- EP-A- 0 402 795
- DE-C- 4 019 299

## Beschreibung

Gegenstand der Erfindung ist ein Verfahren zum quantitativen Auswerten von Agglutinationsreaktionen zwischen einem Reagenz und einer zu untersuchenden biologischen Flüssigkeit. Gegenstand der Erfindung ist ferner eine Vorrichtung zur Durchführung des Verfahrens, die aus einer optischen Meßanordnung aus Lichtquelle, Kondensor, Aufnahmeeinrichtung für ein Meßfeld, Objektiv und Mattscheibe mit optoelektronischer Auswerteeinrichtung besteht.

Agglutinationsreaktionen als Diagnosemethode dienen dem Auffinden und Erkennen bestimmter Krankheiten, wie Myocard-Infarkten, Entzündungen, Rheuma-Faktoren etc. Bei den für die Diagnose erforderlichen Test wird biologische Flüssigkeit, wie Blutserum mit einem spezifischen Antigen auf einer gefärbten Testplatte manuell vermischt. Nach der Reaktion wird das Ergebnis visuell überprüft und diagnostisch ausgewertet. Die Bildung von Agglutinaten läßt dabei auf die Existenz von entsprechenden Antigenen bzw. Antikörpern schließen. Die Signifikanz einer positiven bzw. negativen Reaktion hängt dabei neben den spezifischen biologischen Eigenschaften der eingesetzten Proteine stark von der Beurteilung und der Erfahrung des jeweiligen Experiementators bzw. Laborpersonals ab. Der Nachteil dieser Verfahrensweise liegt darin, daß durch den - aufgrund der Erfahrung des Beobachters - begrenzten Meßbereich in der Regel nur ja/nein-Aussagen über das Resultat der Reaktion möglich sind und keine weitergehenden Aussagen über die Quantifizierung der Reaktion gemacht werden können.

Ferner ist nach der DE-A-39 19 260 ein Verfahren zur quantitativen Auswertung von Agglutinationsreaktionen bekannt, bei dem die gebildeten Agglutinate optoelektronisch erfaßt und über ein festes Zeitintervall integriert werden. Die physikalische Durchmischung der Reaktionspartner innerhalb der Flüssigkeit erfolgt durch Überlagerung einer Taumel- und einer Rotationsbewegung. Während der optoelektronischen Messung ist der Taumelbewegung eine Translationsbewegung überlagert. Das Verfahren gestattet die Messung konzentrationsabhängiger kinetischer Reaktionsabläufe und die Quantifizierung der Agglutinationsreaktion mit Hilfe von entsprechend erstellten Kalibrierkurven. Der Nachteil dieses Meßverfahrens liegt in der Verknüpfung mehrerer komplizierter Bewegungsabläufe zum Durchmischen der Komponenten innerhalb der Flüssigkeit, der Plazierung des Meßgutes in den Strahlengang sowie im Überlagern der Taumelbewegung mit einer Translationsbewegung während der optoelektronischen Messung.

Hier will die Erfindung Abhilfe schaffen. Die Aufgabe wird durch ein Verfahren gelöst, das dadurch gekennzeichnet ist, daß das Reagenz und die biologische Flüssigkeit durch eine horizontale Kreisbewegung innig vermischt werden, danach unter Beibehaltung der Kreisbewegung die im Gemisch gebildeten Agglutinate optisch auf eine Mattscheibe abgebildet werden und das Abbild mit einer elektronischen Auswerteeinrichtung ausgewertet wird.

Zum Durchführen des erfindungsgemäßen Verfahrens wurde ferner eine Vorrichtung der eingangs genannten Art geschaffen, die dadurch gekennzeichnet ist, daß die Aufnahmeeinrichtung aus einer beweglichen Platte besteht, die mit einem Antriebsmotor über eine parallel zur Motorwelle versetzt angeordneten Kupplung und über Verbindungselemente mit einer Unterstützung verbunden ist und die mindestens eine Ausnehmung für ein Meßfeld aufweist.

Die Kupplung kann aus einer auf der Motorwelle angeordneten Scheibe bestehen, die einen exzentrisch angeordneten Stift zum Verbinden von Motor und Platte aufweist.

Das erfindungsgemäße Verfahren ist einfacher als das bekannte Verfahren. Das trifft auch für die Meßvorrichtung zu. Die für die Mischung notwendige Bewegung ist sehr einfach und preisgünstig in einem Meßgerät zu realisieren. Insbesondere lassen sich durch die entsprechende Gestaltung und Lagerung der Aufnahmeeinrichtung für die Meßfelder mehrere Meßfelder darauf anordnen und gleichzeitig erfassen und auswerten. Dadurch können beispielsweise Messungen an unverdünnter Reaktionsflüssigkeit, an einer bis mehrerer Verdünnunosstufen und positive/negative Kontrollmessungen innerhalb eines Meßzyklus gleichzeitig durchgeführt werden. Der optische Abgleich der Meßvorrichtung kann durch Partikel definierter Größe vorgenommen werden. Die Vorrichtung ermöglicht eine genaue und reproduzierbare Bestimmung des Beginns einer Agglutinationsreaktion und die Auszählung der gebildeten Agglutinate auf mehreren Kanälen. So kann beispielsweise bei Verwendung von Antigen, das auf Latex fixiert ist, die untere Nachweisgrenze für Anti-Streptolysin auf 30 IU/ml, für Rheuma-Faktor auf 4 IU/ml und für C-reaktives Protein auf 0,3 mg/dl bestimmt werden.

Bei entsprechender Wahl der Umlauffrequenz und Amplitude von z.B. 1 bis 5 Hz und 5 bis 15 mm der horizontalen Kreisbewegung des Meßfeldes ist eine vollständige Durchmischung der biologischen Flüssigkeit mit dem Antigen bzw. dem Antikörper sowie die Lage des Meßfeldes im Strahlengang der optischen Meßanordnung gewährleistet.

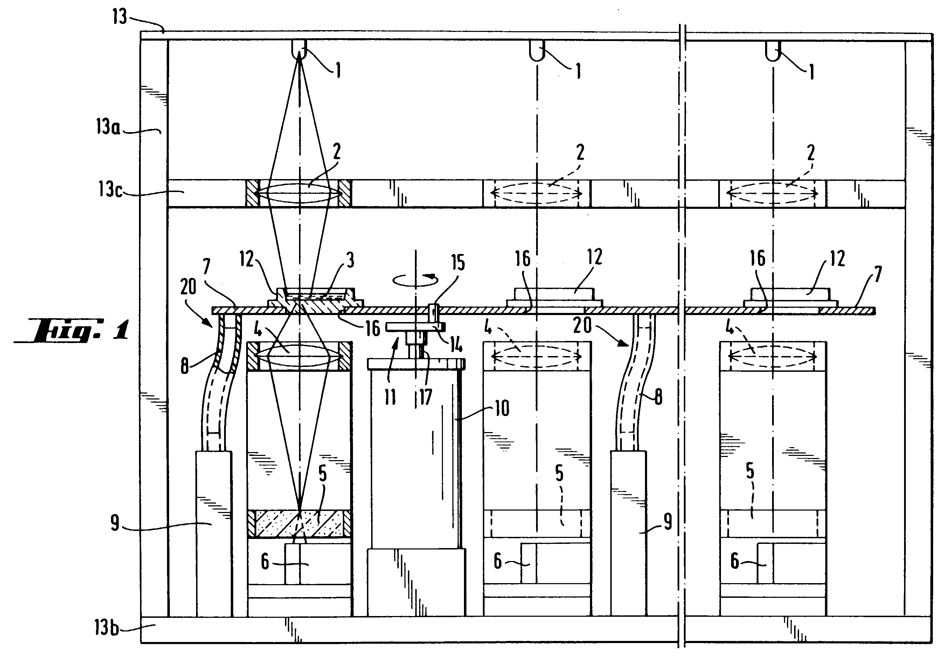

Im folgenden wird die Erfindung anhand der Figuren näher erläutert. Es zeigt
- Figur 1: eine Vorrichtung zum Durchführen des Verfahrens schematisch dargestellt und

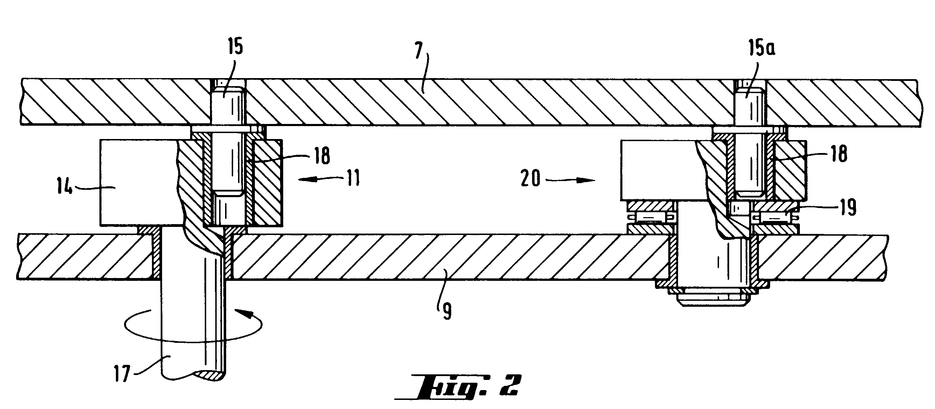

- Figur 2: eine alternative Ausführungsform der Stabilisierung der Aufnahmeeinrichtung für die Meßfelder.

In einem Gestell 13,13a,13b,13c ist eine optische Meßanordnung aus Lichtquelle 1, Kondensor 2, Aufnahmeeinrichtung für das Meßfeld 12, Objektiv 4 und Mattscheibe 5 mit optoelektronischer Auswerteeinrichtung 6 angeordnet. Die Aufnahmeeinrichtung besteht aus einer Platte 7, die mit einem Antriebsmotor 10 über eine Kupplung 11 verbunden ist. Die Kupplung 11 kann aus einer auf der Motorwelle 17 angeordneten Scheibe 14 und einem Stift 15 bestehen, der exzentrisch auf der Scheibe 14 angeordnet und mit der Platte 7 gelenkig verbunden ist. Ferner ist die Platte 7 über Verbindungselemente 20, z.B. über elastische Teile 8 oder über durch Axiallager 19 und Buchsen 18 geführte Stifte 15a mit Unterstützungen 9 verbunden. Die Unterstützungen 9 können aus Stützen oder einer Platte bestehen, die am Gestell 13, 13a, 13b, 13c befestigt sind. Die Platte 7 weist mindestens eine Ausnehmung 16 für ein Meßfeld 12 auf, auf dem sich die Reaktionsflüssigkeit 3, aus biologischer Flüssigkeit und Reagenz befindet. Auf der Platte 7 können mehrere Meßfelder 12, wie angedeutet, angeordnet sein. Als Meßfeld 12 eignen sich transparente Objektträger oder dergleichen. Mit der optoelektronischen Auswerteeinrichtung 6 läßt sich der Reaktionsbeginn, die Gesamtreaktionszeit und die Menge der gebildeten Agglutinate messen.

## Patentansprüche

1. Verfahren zum quantitativen Auswerten von Agglutinationsreaktionen zwischen einem Reagenz und einer zu untersuchenden biologischen Flüssigkeit, dadurch gekennzeichnet, daß das Reagenz und die biologische Flüssigkeit durch eine horizontale Kreisbewegung innig vermischt werden, danach unter Beibehaltung der Kreisbewegung die im Gemisch gebildeten Agglutinate optisch auf eine Mattscheibe abgebildet werden und das Abbild mit einer elektronischen Auswerteeinheit ausgewertet wird.

2. Vorrichtung zum Durchführen des Verfahrens gemäß Anspruch 1, bestehend aus einer optischen Meßanordnung aus Lichtquelle, Kondensor, Aufnahmeeinrichtung für ein Meßfeld, Objektiv und Mattscheibe mit optoelektronischer Auswerteeinrichtung, dadurch gekennzeichnet, daß die Aufnahmeeinrichtung aus einer beweglichen Platte (7) besteht, die mit einem Antriebsmotor (10) über eine parallel zur Motorwelle (17) versetzt angeordnete Kupplung (11) und über Verbindungselemente (20) mit Unterstützungen (9) verbunden ist und die mindestens eine Ausnehmung (16) für ein Meßfeld (12) aufweist.

3. Vorrichtung nach Anspruch 2, dadurch gekennzeichnet, daß die Kupplung (11) aus einer auf der Motorwelle (17) angeordneten Scheibe (14) besteht, die einen exzentrisch angeordneten Stift (15) zum Verbinden von Motor (10) und Platte (7) aufweist.

4. Vorrichtung nach Anspruch 2, dadurch gekennzeichnet, daß die Unterstützung (9) aus einer mit einem Gestell (13, 13a, 13b, 13c) verbundenen Platte besteht, auf der sich die beweglichen Platte (7) über durch Axiallager (19) geführte Stifte (15a) abstützt.

## Claims

1. A method for quantitative evaluation of agglutination reactions between a reagent and a biological fluid to be examined, wherein the reagent and the biological fluid are intimately mixed by a horizontal circular movement, the agglutinates formed in the mixture while the circular movement is maintained are optically imaged thereafter on a ground glass plate, and the image is evaluated using an electronic evaluation device.

2. An apparatus for carrying out the method as claimed in claim 1, composed of an optical measuring arrangement of a light source, condenser, detecting device for a measuring field, objective and ground glass plate with an optoelectronic evaluating device , wherein the detecting device is composed of a movable plate (7) which is connected to a drive motor (10) via a coupling (11) arranged offset parallel to the motor shaft (17), and to supports (9) via connecting elements (20), and which has at least one cutout (16) for a measuring field (12).

3. The apparatus as claimed in claim 2, wherein the coupling (11) is composed of a disk (14), arranged on the motor shaft (17), which has an eccentrically arranged pin (15) for connecting the motor (10) and plate (7).

4. The apparatus as claimed in claim 2, wherein the support (9) is composed of a plate that is connected to a frame (13, 13a, 13b, 13c) and on which the movable plate (7) is supported via pins (15a) guided through thrust bearings (19).

## Revendications

1. Procédé d'évaluation quantitative de réactions d'agglutination entre un réactif et un liquide biologique à étudier, caractérisé en ce que le réactif et le liquide biologique sont mélangés intimement l'un à l'autre par un mouvement circulaire horizontal, qu'ensuite, tout en maintenant le mouvement circulaire, une image des agglutinats formés dans le mélange est projetée optiquement sur une plaque dépolie et l'image est évaluée à l'aide d'une unité d'évaluation électronique.

2. Dispositif pour la mise en oeuvre du procédé selon la revendication 1, constitué par un dispositif de mesure optique composé d'une source de lumière, d'un condenseur, d'un dispositif récepteur destiné à recevoir un champ de mesure, d'un objectif et d'une plaque dépolie munie d'un dispositif d'évaluation opto-électronique, caractérisé en ce que le dispositif récepteur est constitué par une plaque mobile (7) qui est reliée à un moteur d'entraînement (10) par un accouplement (11) déporté parallèlement à l'arbre (17) du moteur, et reliée à des supports (9) par des éléments de liaison (20) et qui présente au moins un évidement (16) Pour un champ de mesure (12).

3. Dispositif selon la revendication 2, caractérisé en ce que l'accouplement est constitué par un disque (14) monté sur l'arbre (17) du moteur et qui présente un tourillon (15) disposé excentriquement et servant à relier le moteur (10) à la plaque (7).

4. Dispositif selon la revendication 2, caractérisé en ce que le support (9) est constitué par une plaque reliée à un bâti (13, 13a, 13b, 13c) sur laquelle la plaque mobile (7) prend appui par l'intermédiaire de tourillons (15a) guidés par des paliers axiaux (19).
